(19) Europäisches Patentamt
European Patent Office
Office européen des brevets

(11) **EP 4 322 850 B1**

(12) **EUROPEAN PATENT SPECIFICATION**

(45) Date of publication and mention
of the grant of the patent:
**01.04.2026  Bulletin 2026/14**

(21) Application number: **22721371.7**

(22) Date of filing: **08.04.2022**

(51) International Patent Classification (IPC):
**A61B 5/12** *(2006.01)*

(52) Cooperative Patent Classification (CPC):
**A61B 5/12**

(86) International application number:
**PCT/EP2022/059397**

(87) International publication number:
**WO 2022/218846 (20.10.2022 Gazette 2022/42)**

(54) **A HEARING ESTIMATION SYSTEM**

HÖRSCHÄTZUNGSSYSTEM

SYSTÈME D'ESTIMATION D'AUDITION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.04.2021  DK PA202100378**

(43) Date of publication of application:
**21.02.2024  Bulletin 2024/08**

(73) Proprietor: **WS Audiology A/S
3540 Lynge (DK)**

(72) Inventors:
• **OUGAARD, Asger
  3540 Lynge (DK)**
• **JESPERSEN, Caspar Aleksander Bang
  3540 Lynge (DK)**

(56) References cited:
**WO-A1-2019/195866      WO-A1-2020/214482
US-A1- 2016 345 111**

• **OÊZDAMAR Ö ET AL: "Classification of
audiograms by sequential testing using a
dynamic Bayesian procedure", THE JOURNAL
OF THE ACOUSTICAL SOCIETY OF AMERICA, 1
January 1990 (1990-01-01), Woodbury, NY, pages
2171 - 2179, XP055945412, Retrieved from the
Internet <URL:https://asa.scitation.org/doi/pdf/
10.1121/1.400114> [retrieved on 20220722], DOI:
10.1121/1.400114**

**Description**

The present invention relates to a hearing estimation system.

BACKGROUND OF THE INVENTION

Generally, a hearing aid system according to the invention is understood as meaning any system which provides an output signal that can be perceived as an auditory signal by a user or contributes to providing such an output signal, and which has means adapted to compensate for an individual hearing loss of the user or contribute to compensating for the hearing loss of the user. These systems may comprise hearing aids that can be worn on the body or on the head, in particular on or in the ear, or that can be fully or partially implanted. However, a device whose main aim is not to compensate for a hearing loss, for example a consumer electronic device (mobile phones, MP3 players, so-called "hearables" etc.), may also be considered a hearing aid system, provided it has measures for compensating for an individual hearing loss.

Within the present context, a hearing aid can be understood as a small, batterypowered, microelectronic device designed to be worn behind or in the human ear by a hearing-impaired user. Prior to use, the hearing aid is adjusted by a hearing aid fitter according to a prescription. The prescription is based on a hearing test, resulting in a so-called audiogram, of the performance of the hearing-impaired user's unaided hearing. The prescription is developed to reach a setting where the hearing aid will alleviate a hearing loss by amplifying sound at frequencies in those parts of the audible frequency range where the user suffers a hearing deficit. A hearing aid comprises one or more microphones (or, more generally, electroacoustic input transducers), a battery, a microelectronic circuit comprising a signal processor, and an acoustic output transducer. The signal processor is preferably a digital signal processor. The hearing aid is enclosed in a casing suitable for fitting behind or in a human ear.

According to variations, the hearing aid need not comprise a traditional loudspeaker as output transducer. Examples of hearing aid systems that do not comprise a traditional loudspeaker are cochlear implants, implantable middle ear hearing devices (IMEHD), bone-anchored hearing aids (BAHA) and various other electro-mechanical transducer-based solutions including, e.g., systems based on using a laser diode for directly inducing vibration of the eardrum.

Within the present context a hearing aid system may comprise a single hearing aid (a so-called monaural hearing aid system) or comprise two hearing aids, one for each ear of the hearing aid user (a so-called binaural hearing aid system).

In a traditional hearing aid fitting, the hearing aid user goes to a site of a hearing aid fitter (e.g., an acoustician), and the user's hearing aids are adjusted using the fitting equipment that the hearing aid fitter has in his office. Typically, the fitting equipment comprises a computer capable of executing the relevant hearing aid programming software and a programming device adapted to provide a link between the computer and the hearing aid or some other audio device comprising an acoustic output transducer.

The hearing loss of a hearing-impaired person is generally frequency-dependent and may not be the same for both ears. This means that the hearing loss of the person varies depending on the frequency. Therefore, when compensating for hearing losses, it can be advantageous to utilize frequency-dependent amplification. Hearing aids therefore often provide band split filters in order to split an input sound signal received by an input transducer of the hearing aid, into various frequency intervals, also called frequency bands, which are independently processed. In this way it is possible to adjust the input sound signal of each frequency band individually to account for the hearing loss in respective frequency bands.

The frequency dependent adjustment is normally done by implementing a band split filter and a compressor for each of the frequency bands, hereby forming so-called band split compressors, which may be combined to form a multi-band compressor. In this way, it is possible to adjust the gain individually in each frequency band depending on the hearing loss as well as on the input level of the input sound signal in a respective frequency band. For example, a band split compressor may provide a higher gain for a soft sound than for a loud sound in each frequency band.

Traditionally a hearing aid system is fitted - initially or as part of a subsequent fine tuning - based primarily or exclusively on a recorded audiogram for the individual haring aid system user.

Perhaps the most widespread method is based on pure tone tests, where the individual to be tested (i.e. the test person) is presented for a tone at a specific frequency and at first at a very low loudness that most probably is not audible for the test person, where after the loudness is progressively increased until the test person indicates that the tone is audible whereby the hearing threshold may be established, and from that the hearing loss at that specific frequency as compared to normal hearing subjects may be derived. In order to fully characterize the hearing loss, the test may be repeated for other frequencies in the audible range.

This type of test has been offered as online test for many years. Hereby, an individual who suspects possibly having a hearing loss can take the test at home and record their audiogram without having to make an appointment and travel to a hearing care professional.

Known methods of audiogram analysis are shown in Oêzdamar Ö ET AL: "Classification of audiograms by sequential testing using a dynamic Bayesian procedure", The Journal of the Acoustical Society of America, 1 January 1990, Woodbury, NY, DOI: 10.1121/1.400114, WO2019/195866-A1, US2016/345111-A1, and WO2020/214482-A1.

However, this type of test may be time consuming and some users consider the test uncomfortable and annoying, which additionally may lead to a recorded audiogram of low accuracy.

It is therefore an object of the present invention to provide an improved method of quickly estimating an audiogram, which preferably shall be comfortable for the test person.

SUMMARY OF THE INVENTION

According to one aspect of the invention, this object is solved by a hearing estimation system according to claim 1.

According to another aspect of the invention, this object is solved by a non-transitory computer readable medium according to claim 14.

DETAILED DESCRIPTION OF THE INVENTION

The first audiograms may be given by audiograms measured by an audiometer, or may also be given by in-situ audiograms, i.e., by audiograms measured in the presence of a hearing aid in or at the respective ear during measurement. In the following, both kinds of audiograms may be comprised, except as otherwise stated by explicit specification. For each person out of the first plurality, a corresponding first audiogram is provided, hence the corresponding first plurality of first audiograms. The persons of the first plurality, preferably are given by hearing impaired persons; however, also persons without any noticeable hearing impairment may be included, in particular for statistical reasons.

Preferably, each of the first audiograms is representative of the corresponding person's hearing threshold at several frequencies, wherein the first audiograms may also contain interpolations between the measured hearing thresholds. Most preferably, the frequencies at which a respective hearing threshold is measured for generating the audiogram are equal for all persons of the first plurality.

To each of the first audiograms, a weighting factor is associated, and a respective initial value for each weighting factor is provided, i.e., a number of weighting factors corresponding to the first plurality is initialized by said initial values. In a preferred embodiment, the initial values are equal for all weighting factors associated to the different first audiograms. However, different initial values for different first audiograms are also possible, e.g., to include further statistical information.

Preferably, the first set of frequencies corresponds to the measurement frequencies at which the hearing threshold is determined for obtaining the first audiograms. The first frequency may be selected as a fixed frequency, e.g., as a frequency of 1 kHz or as close as possible to 1 kHz within the first set of frequencies, or may also be selected dynamically, by assigning some spread measure to the first audiograms, and evaluating the spread measure in a way such that a possible deviation from the first audiograms may be reduced the most by a corresponding dynamical determination of the first frequency, a corresponding hearing threshold measurement at said dynamically determined first frequency and a respective update of the weighting factors. The first frequency may furthermore be determined based on further information, such as statistical and/or demographic and/or audiological information about the persons corresponding to the first audiograms.

Determining the specific user's estimated audiogram as a weighted mean based on the weighting factors may in particular comprise using directly the respective values of the weighting factors given by their first update. However, and in particular in case of further iterations, the information contained in the first update of the weighting factors is also used for further updates of the weighting factors, thus the notion of a weighted mean based on the weighting factors according to the first update shall also include this case of further updates.

In an advantageous way, the method extracts and uses statistical information contained in the first audiograms, in order to reduce and possibly minimize the number of frequencies at which a true hearing threshold measurement of the specific user needs to be performed in order to obtain a reliable audiogram of said specific user. The first frequency may be selected in a way such that a single hearing threshold measurement at the selected frequency in order to reduce an "a priory uncertainty" about the underlying true audiogram, and thus, the full range hearing ability of the specific user, is performed where said measurement may yield the most relevant information gain with respect to the true audiogram.

In an embodiment, the method further comprises a step of determining, at each of said first set of frequencies, a weighted variance metric for said first plurality of first audiograms, in dependence of said weighting factors, wherein the first frequency is selected out of said first set of frequencies by a determination in dependence on the frequency out of a first frequency range for which the weighted variance metric is largest.

The notion of a weighted variance metric for the first plurality of audiograms, at a given frequency out of the first set of frequencies, shall be understood as a metric for the dispersion or the spread of the different first audiograms' values at the given frequency of interest, satisfying the conditions of a variance. In particular, the weighted variance metric is given by the weighted sum of squared mean deviations of all the first audiograms at the given frequencies, the weighted sum being performed according to the respective initial values of the weighting factors.

The determination of the first frequency, out of said first set of frequencies, in dependence on the frequency out of a first frequency range for which the weighted variance metric is largest, in particular may comprise the case that the first frequency does not equal said frequency for which the weighted variance metric is largest in the first frequency range, e.g., in case of interpolations and/or continuous fittings of the first audiograms and/or of the weighted variance metric over the first set of frequencies. In a preferred embodiment, the first frequency is chosen as the frequency for which the weighted variance metric is largest.

The first frequency range may be chosen such that the first frequency is chosen out of a subset of the first set of frequencies, said subset covering the first frequency range, in order to ensure that the first frequency is chosen in a relevant frequency range from an audiological point of view, e.g., from 500 Hz to 3 kHz, preferably from 1 kHz to 2,5 kHz, or similar. Then, even if the weighted variance metric of the first audiograms may be larger for some frequency out of the first set of frequencies, the first frequency is chosen as the frequency with the maximal weighted variance metric over the first frequency range. However, this condition may also be relaxed, and the first frequency range may also cover the full frequency range, such that the first frequency is chosen in dependence on the frequency of the overall maximum of the weighted variance metric (over all frequencies).

Since the first frequency is determined based on the spread of the first audiograms, it may be selected in a way such that this spread, i.e., the "a priory uncertainty" about the underlying true audiogram, and thus, the full range hearing ability of the specific user, is reduced the most at the selected frequency by a single hearing threshold measurement.

In a preferred embodiment, the method comprises an iteration of the following steps: determining, at several frequencies out of said first set of frequencies, a weighted variance metric for said first plurality of first audiograms in dependence on said weighting factors, according to their most recent update, respectively; determining a distinguished frequency out of said several frequencies, also in dependence on and preferably as the frequency out of a second frequency range for which said weighted variance metric is largest; measuring a hearing threshold of said specific user at said distinguished frequency; performing an update on each of said weighting factors corresponding to said first plurality of first audiograms in dependence on the difference between the value of the respective first audiogram and said measured hearing threshold of said specific user at said distinguished frequency, respectively; and, after finishing said iteration, further comprising the step of determining the estimated audiogram for said specific user as a weighted mean of said first plurality of first audiograms, using said weighting factors according to their most recent update, respectively, for said weighted mean.

The second frequency range may be given by the first frequency range. In particular, the second frequency range may be given by the full frequency range. In case that the first frequency range is not given by the full frequency range, this in particular means that the first frequency is determined from the maximum frequency of the weighted variance metric over a limited frequency range (the first frequency range), and the first update is performed. Afterwards, the distinguished frequency is determined from the overall maximum frequency of the (updated) weighted variance metric.

Preferably, in each iteration run, the weighted variance metric is determined at all frequencies out of said first set of frequencies for which no hearing threshold of said specific user has been measured yet. In particular, this may include that the number of frequencies at which the weighted variance metric has to be determined for the first audiograms, may reduce with each iteration run. The determination of the distinguished frequency, in particular, may be performed in an analogous way to the determination of the first frequency. Thus, conditions and possible embodiments may be transferred from said determination of the first frequency, mutatis mutandis.

Likewise, the update of the weighting factors corresponding to the first audiograms in dependence on the difference between the value of the respective first audiogram and said measured hearing threshold of said specific user at said distinguished frequency, may be performed in an analogous way to the update in dependence on the difference between the value of the respective audiogram and the specific user's measured hearing threshold at the first frequency. Preferably, for each weighting factor, the update may also depend on each of the differences between the respective values of the first audiograms and the hearing thresholds of the specific user already measured before, at their respective frequencies, i.e., at the first frequency, and possibly at distinguished frequencies during earlier iteration runs.

Iterating the procedure increases the accuracy of the audiogram estimation while still allowing for a reduction of hearing threshold measurements. Typically, an audiogram contains hearing threshold measurement at about 8 to 10 frequencies, so an iteration of two or three total runs (including the initial run corresponding to the first frequency) provides a good tradeoff between an optimal accuracy of the audiogram and a quick determination.

In an embodiment, the iteration is finished after completion of a given number of iteration runs or when the weighted variance metric for said first plurality of first audiograms or a variance for said first plurality of first audiograms falls below a given first threshold. Finishing after a given number of total iteration runs - e.g., after the second or third update to the weighting factors and the respective estimation of the specific user's audiogram - ensures that the estimation has a controllable duration and complexity. Finishing the estimation based on a threshold on the weighted variance metric - according to the most recent update to the weighting factors - or on the total variance of the first audiograms allows for ensuring a lower bound on the estimation accuracy.

Preferably, after each iteration run, the estimated audiogram for said specific user is determined as a weighted mean of said first plurality of first audiograms, using said weighting factors according to their most recent update, respectively, for said weighted mean, the estimated audiogram is visualized, and said visualized estimated audiogram presented to a hearing care professional, such as an acoustician or an audiologist, for decision about stopping the iteration. The visualization is preferably performed by means of an appropriate apparatus comprising a screen or a similar displaying device. E.g., a computerized device on which all processing steps of the method are implemented may either comprise a screen or a similar displaying device for visualization, or may be operationally connected to a screen or a similar displaying device. The computerized device may also comprise - and/or be operationally connected to - suitable input means, such as a keyboard or a touch screen, for the hearing care professional to enter a decision to stop the iteration. It can be of advantage for a hearing care professional who is performing the fitting to decide on when the audiogram estimation is sufficiently accurate.

In an embodiment, for each weighting factor, the initial value is chosen in dependence on a demographic similarity of the specific user with the person corresponding to the first audiogram for which the weighting factor is being provided. The demographic similarity may be with respect to a gender and/or an age group and/or an ethnic group and/or a socioeconomic group (e.g., "working class", "white collar" etc.) and/or a residential settlement type (e.g., "urban", "rural" etc.). It can be of advantage to assign a stronger weight to first audiograms from persons with a similar demographical background as the specific user, such as a similar age or a similar working background (and thus, an assumed similarity in previous noise exposure).

In an embodiment, the first plurality of first audiograms is chosen as a subset out of a multiplicity of first audiograms from a corresponding multiplicity of preferably hearing impaired persons in dependence on a demographic similarity of the specific person with a person from said multiplicity corresponding to a respective first audiogram. This means in particular that there exists an overall database of first audiograms (the multiplicity), and for the estimation of the specific user's audiogram, the first plurality of audiograms is selected out of the multiplicity by using demographic criteria, in an analogous way as explained above, in order to have first audiograms from persons who are sufficiently "similar" to the specific user with respect to their demographics. This way, it can be assumed that the spread of first audiograms is reduced, and the accuracy of the estimation may be increased even for performing the method by a single run or few iteration runs.

In an embodiment, a starting sound pressure level (SPL) for measuring the hearing threshold of said specific user at a certain frequency is chosen in dependence on the estimated audiogram and/or weighted variance metric at said certain frequency, using the weighting factors according to their most recent update, respectively. Said certain frequency, in particular, may be given the first frequency or by a distinguished frequency during a subsequent iteration run. Typically, in order to measure a hearing threshold of a test person at a given frequency, a probe tone such as a sine tone is produced with a SPL sufficiently high to ensure that even a hearing impaired test person will normally perceive the probe tone. Then, the SPL is slowly reduced (continuously or step-wise) in order to reach the level at which the test person ceases to hear the probe tone. However, adjusting the SPL to the statistics obtained for a certain frequency from the weighted variance metric allows for a faster measurement of the hearing threshold, as SPL ranges where close to everybody (in particular, persons with a similar hearing impairment as the specific user, due to their stronger weighting) is still hearing, may be skipped.

In an embodiment, a continuous fit is performed on the weighted variance metric for all frequencies in the interval spanned by said first set of frequencies, wherein the first frequency is determined from said continuous fit. In particular, this means: the first set of frequencies, as a discrete set, spans a frequency interval. At all frequencies in the first set, the weighted variance metric for the first audiograms is determined, yielding discrete data points in frequency space. Then, a continuous fit such as a polynomial fit is performed over these discrete data points, yielding a curve of a continuous variance function with a continuous frequency dependence.

The first frequency may then be derived from said continuous variance function, e.g., as the frequency where said continuous variance function attains its maximum value.

Preferably, in order to obtain a value of a first audiogram at the first frequency, an interpolation and/or a continuous fit is performed on basis of said first audiogram's values at the frequencies of said first set of frequencies. In particular, the first audiograms may be interpolated (e.g., by a linear or logarithmic interpolation of a first audiogram's values between two frequencies) or continuously fit in a way similar to the weighted variance metric in order to obtain values for each of the first audiograms at frequencies not comprised in the first set of frequencies, and in particular, at which no measured values exist for some or any of the first audiograms. This way, values for the first audiograms may also be obtained in case the first frequency is not comprised in the first set of frequencies.

In an embodiment, the method further comprises the steps of: providing a second plurality of second audiograms from a corresponding second plurality of preferably hearing impaired persons, wherein each person out of the first plurality is also comprised in the second plurality of persons; providing, for each of the second audiograms, a value of a weighting factor; determining, at each of a second set of frequencies, a weighted variance metric for said second plurality of second audiograms, in dependence of said weighting factors, wherein the second set of frequencies is a subset said first set of frequencies; and determining said first frequency and/or said distinguished frequency for the first plurality of first audiograms and/or performing said first update on the weighting factors corresponding to the first audiograms also based on the weighted variance metric for said second plurality of second audiograms.

The second set of frequencies may be a proper subset of said first set of frequencies (i.e., there are frequencies contained in the first set but not in the second set of frequencies) or a so-called improper subset (then, the first set and the second set comprise the same frequencies). The first plurality of persons may be equal to the second plurality of persons, or the second plurality of persons may be larger than the first plurality (i.e., some persons of the second plurality are not comprised in the first plurality). The second audiograms may be of the same nature as the first audiograms, or the first audiograms may in particular given by in-situ audiograms, while the second audiograms may be given by "conventional" audiograms.

The method is then partially performed on the second audiograms in the sense that for the second set of frequencies, a weighted variance metric for the second audiograms is provided. The information contained in said weighted variance metric for the second audiograms may then be used to determine the first frequency and/or the distinguished frequency for the first audiograms.

In particular, the first and second pluralities of preferably hearing impaired persons comprise the same persons, wherein for each person out of said first and second pluralities, respectively, the corresponding first and second audiogram are representing the hearing at either of the two ears. This particularly means that the first and second audiograms are measured at the left and right ear (no specific assignment about which audiogram corresponds to which ear shall be implied) of each person out of the first plurality of persons (which are the same persons as the second plurality). Even though hearing loss normally affects both ears of a hearing impaired person in a different way, the ensemble statistics inherent in the second audiograms (corresponding to one respective ear of a group of persons) may be used in order to decrease uncertainty about the first audiograms (corresponding to the respective other ear of said group of persons). E.g., for the second set of frequencies, the similarities of a user's hearing threshold, measured at one ear, to the second audiograms (given in terms of the weighted mean and/or the respective weighted variance metric) may be used in order to determine initial values for the weighting factors for the first audiograms corresponding to the other ear.

In another embodiment, each of the first audiograms is measured as an in-situ audiogram in the presence of a hearing aid at the respective ear of the corresponding person out of the first plurality, and each of the second audiograms is measured in the absence of a hearing aid at the respective ear of the corresponding person out of the second plurality. This particularly means that the first audiograms, as well as preferably the audiogram to be estimated for the specific user, are in-situ audiograms, measured with a hearing aid located on or at or in the ear of the person corresponding to a respective first audiogram during measurement of the hearing threshold. The second audiograms, in particular, are given by "conventional" audiograms measured, e.g., by an audiometer or with help of a electroencephalography measurement, without any hearing device in or on or at the ear during the measurement. The second plurality of persons may comprise more persons than the first plurality, as it is typically easier to get conventional audiograms, while it is more difficult to get in-situ audiograms. So, this way, the data scarcity for the first, in-situ audiograms (and the statistical drawbacks resulting therefrom) may be compensated for by using also statistical information from the "conventional" second audiograms (which are better available), and in particular, using the intrinsic correlation for the persons comprised both in the first and second plurality. E.g., for the second set of frequencies, the similarities of a user's hearing threshold, measured as a "normal audiogram", to the second audiograms (given in terms of the weighted mean and/or the respective weighted variance metric) may be used in order to determine initial values for the weighting factors for the first audiograms in order to estimate a user's in-situ audiogram.

The invention furthermore discloses a hearing estimation system comprising a computerized device and a hearing aid, wherein the computerized device is operationally connected to the hearing aid and wherein the computerized device comprises a graphical user interface, a program storage for storing an executable program and a processor for executing said program to perform the method according to one of the preceding claims. The system according to the invention shares the benefits of the method according to the invention. The advantages of the proposed method and of its preferred embodiments can be transferred to the system itself in a straight forward manner. The computerized device may in particular comprise a computer with a CPU and a RAM addressable via said CPU, configured to implement the mathematical operations of the method on the first (and possibly second) audiograms and their respective weighting factors, and further comprising suitable means for measuring a hearing threshold of the specific user, such as, e.g., an audiometer.

However, the hearing estimation system needs not comprise a hearing aid, instead it may comprise some other audio device (i.e. a device comprising an acoustic output transducer) without means for hearing loss compensation or at least be operationally connected to such an audio device.

The computerized device may be a smart phone or a personal computer. Furthermore the computerized device may comprise two parts such as a smart phone and a server in the cloud, whereby at least some of the more resource demanding processing can be distributed to e.g. a server in the cloud where these resources are more abundant.

BRIEF DESCRIPTION OF THE DRAWINGS

The attributes and properties as well as the advantages of the invention which have been described above are now illustrated with help of drawings of an embodiment example. In detail,

figure 1    shows, in a schematic block diagram, a method for estimating an audiogram of a specific user with help of audiograms of other persons,

figure 2    shows, in a series of SPL over frequency diagrams, the results of an iteration of the method according to figure 2, and

figure 3    shows, in a schematic block diagram, the method according to figure 1, additionally using a second data set of different audiograms for the estimation.

Parts and variables corresponding to one another are provided with the same reference numerals in each case of occurrence for all figures.

DETAILED DESCRIPTION OF THE DRAWINGS

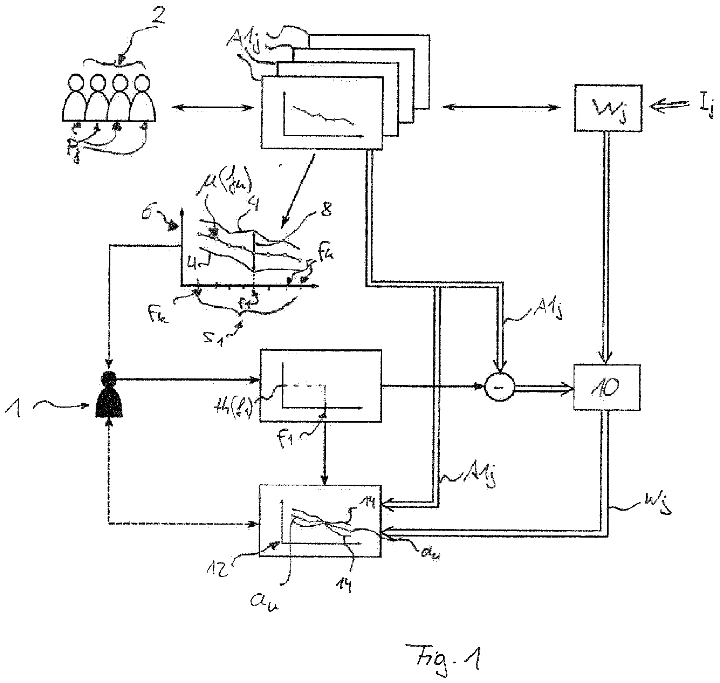

In figure 1, a schematic block diagram is shown, said block diagram displaying the workflow of a method for estimating an audiogram of a specific user 1. The specific user 1, in particular, may be a hearing impaired person which typically uses a hearing aid (not shown) for correcting his hearing loss. The audiogram to be estimated in a way to be described above, in particular, may be used for fitting the hearing aid of the specific user 1 to his individual audiological needs.

In order to estimate an audiogram for the specific user 1, a first plurality of first audiograms A1j is provided from a database, each of which corresponding to a hearing impaired person $P_j$ out of a first plurality 2 of persons. This means in particular that a database with first audiograms A1j, taken from hearing impaired persons $P_j$ via respective hearing threshold measurements over frequency, are given, said hearing impaired persons $P_j$ amounting to the first plurality 2 of persons.

Furthermore, a corresponding weighting factor $w_j$ is assigned to each of the first audiograms A1j, and an initial value $I_j$ for each of these weighting factors $w_j$ is provided. In other words, a relationship is established between each hearing impaired person $P_j$, his/her first audiogram A1j, the associated weighting factor wj and the initial value $I_j$ for said weighting factor $w_j$. In the following, this direct relationship will be explicitly used.

When no further knowledge on the hearing impaired persons $P_j$ is used, the initial values $I_j$ may be provided equal for all weighting factors $w_j$, i.e., $w_j = 1 \, \forall \, j$ (equivalently, $w_j = 1/N$ may be used $\forall j$, being N the total number of first audiograms A1j). However, it is also possible to provide different initial values $I_j$, in dependence on demographic resemblance of a hearing impaired person $P_j$ with the specific user 1. In particular, a higher or lower initial value $I_j$ may be provided for weighting factors $w_j$ corresponding first audiograms A1j from hearing impaired persons $P_j$ out of the same or out of different age groups, working background groups, residential settlement type etc. The differences to be provided in the initial values $I_j$ according to the demographic grouping may be obtained from statistical considerations of the first audiograms A1a with respect to said demographic grouping. For example, the initial value $I_j$ for a weighting factor $w_j$ may be set to $I_j = 1$ for all hearing impaired persons $P_j$ out of the same 10-year age-group as the specific user 1, and $I_j = 0.8$ for hearing impaired persons $P_j$ out of a neighboring 10-year age-group, $I_j = 0.6$ for the next neighboring 10-year age-group and so on. Furthermore, the first plurality 2 of persons $P_j$ may be chosen out of a multiplicity of persons (not shown) which is a superset to the first plurality 2 of persons $P_j$, to which respective first audiograms A1j are available, in dependence on demographic similarity of the persons $P_j$ with the specific user 1. Thus, the first plurality of first audiograms A1j for estimating the audiogram of the specific user 1 is chosen out of the multiplicity of the first audiograms A1j each of which corresponding to one out of said multiplicity of persons.

For each frequency fk out of a first set $s_1$ of frequencies, a weighted variance metric $v_k \, (w_j)$ for the first audiograms A1j is determined in dependence on the corresponding weighting factors $w_j$. The weighted variance metric $v_k \, (w_j)$, in particular, at each frequency $f_k$ may be calculated as a weighted sum of the squared deviations of the respective values of the first audiograms A1j from their weighted mean $\mu$, at that frequency:

$$v_k(w_j) = \frac{1}{\sum_k w_k} \sum_j^N w_j \cdot [A1j(f_k) - \mu(f_k)]^2$$

(i)

with

$$\mu(f_k) = \frac{1}{\sum_k w_k} \sum_j^N w_j \cdot A1j(f_k),$$

(ii)

and N being the total number of first audiograms A1j (i.e., the cardinality of the first plurality 2 of persons). Hereby, the argument $w_j$ of $v_k$ shall indicate that the weighted variance metric $v_k$ depends on the particular values of the weighting factors $w_j$. In case that the initial values $l_j$ for the weighting factors $w_j$ have been chosen to $l_j = 1 \, \forall \, j$, then the weighted variance metric $v_k$ yields the normal variance.

Preferably, the first audiograms A1j always show hearing threshold measurements of the underlying hearing impaired persons $P_j$ at the same set of frequencies, i.e., the first set $s_1$ of frequencies $f_k$. However, in case that there are first audiograms A1j which do not have a hearing threshold measurement at one or several frequencies $f_k$ out of the first set $s_1$ of frequencies (they may have hearing threshold measurements at alternative frequencies), a linear interpolation or a continuous fit (e.g., a polynomial fit) may be performed on each of these first audiograms A1j, based on the frequencies $f_k$ out of the first set $s_1$ of frequencies at which these audiograms do have hearing threshold measurement data.

The weighted variance metric $v_k$ is indicated in figure 1 by a variance corridor 4 around the mean $\mu(f_k)$ in a respective diagram 6. The variance corridor 4 can be understood as a corridor with a width (i.e., a height in figure 1) of $\pm v_k$ around the mean $\mu(f_k)$ at each frequency $f_k$, and shall illustrate the inherent uncertainty for the mean $\mu(f_k)$ that the weighted variance metric $v_k$ represents at each frequency $f_k$. Now, a first frequency $f_1$ is determined as the frequency $f_k$ out of the first set $s_1$ of frequencies $f_k$ at which the weighted variance metric $v_k$ has the largest value (indicated by the width 8 for the variance corridor 4 at the first frequency $f_1$). In alternative embodiments (not shown), the first frequency may also be determined as a frequency out of a frequency range spanning a true subset of the first set $s_1$ of frequencies $f_k$ at which the weighted variance metric $v_k$ has the largest value, or may also be selected as a fixed frequency, e.g., as $f_1 = 1$ kHz.

Now, a hearing threshold th of the specific user 1 is measured at the first frequency $f_1$, i.e., th$(f_1)$. This can be done, e.g., by audiometry or similar methods. In general, a probe sound signal of a known SPL deemed sufficiently high for the specific user 1 hearing said probe sound signal is being presented to the hearing of the specific user 1, and the SPL is decreased until the specific user 1 indicates that he cannot hear the probe sound signal anymore.

Then, for each of the first audiograms A1j, the corresponding weighting factor wj - still being at its respective initial value $l_j$ - receives a first update 10. This first update 10 is performed for each weighting factor on the basis of the difference A1j$(f_1)$ - th$(f_1)$, i.e., in dependence on the difference of the first audiogram A1j (corresponding to the weighting factor $w_j$ to be updated), taken at the first frequency $f_1$, and the measured hearing threshold th of the specific user 1 at the first frequency $f_1$. The first update 10 is preferably done in a way that for A1j$(f_1)$ = th$(f_1)$, the weighting factor $w_j$ attains the maximal possible value, and for an increasing difference, A1j$(f_1)$ - th$(f_1)$, the weighting factor wj decreases more and more, as a result of the first update 10.

After the first update 10, the weighting factors wj are used to generate an estimated audiogram au for the specific user 1, by means of a weighted sum of the first audiograms A1j, as

$$a_u(f_k) = \frac{1}{\sum_k w_k} \sum_j^N w_j \cdot A1_j(f_k).$$

(iii)

Note that the estimated audiogram $a_u(f_k)$ as given in eq. (iii) corresponds to the weighted mean $\mu(f_k)$ as given in eq. (ii) (with the particular exception that the estimated audiogram $a_u$ is being calculated with the updated weights $w_j$.

In figure 1, the estimated audiogram $a_u$ for the specific user 1 is shown in a diagram 12 compared to the variance corridor 14. Preferably, the weighting factor $w_j$ according to the first update 10 shall satisfy the condition that for the first

frequency $f_1$, the estimated audiogram au for the specific user 1 as given in eq. (iii) exactly reproduces the measured hearing threshold th $(f_1)$, i.e., $th(f_1) \equiv \frac{1}{\sum_k w_k} \sum_j^N w_j \cdot A1_j(f_1)$. If this is not the case, then the estimated audiogram au $(f_1)$ for the first frequency $f_1$ may also be set to be equal to the measured hearing threshold th $(f_1)$ at the first frequency $f_1$ as a design choice, i.e., the value au $(f_k)$ as indicated in eq. (iii) is taken only for frequencies $f_k \neq f_1$. The estimated audiogram au be used directly for an individual fitting of a hearing aid of the specific user 1.

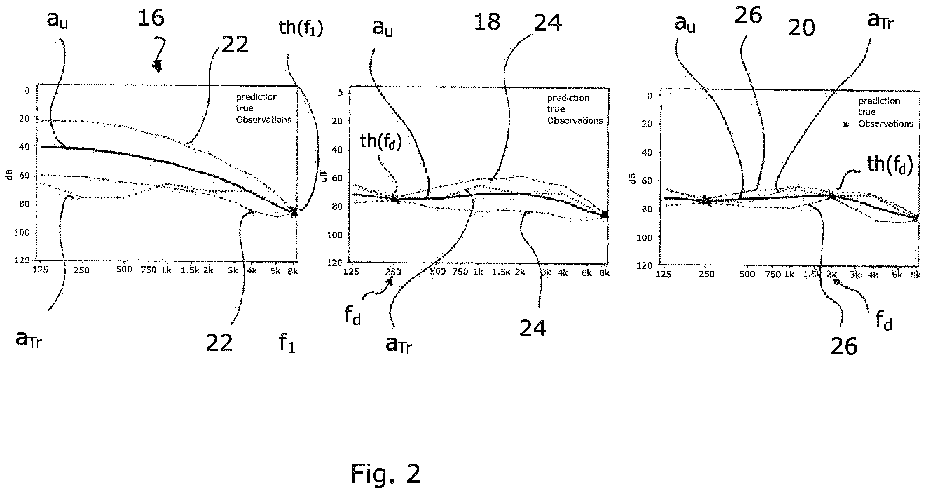

However, the process shown in figure 1 may also be iterated, thus generating an estimated audiogram au with a higher accuracy. This is displayed with help of figure 2, a series of three SPL over frequency diagrams 16, 18 and 20 are shown.

In each of the diagrams 16, 18, 20, the progress of an iteration of the method shown in figure 1 is displayed by means of the estimated audiogram au resulting from the respective iteration at a first set $s_1$ of frequencies $f_k$ of 125 Hz, 250 Hz, 500 Hz, 1kHz, 2 kHz, 3 kHz, 4 kHz, 6 kHz and 8 kHz. For each of these frequencies, the estimated audiogram au, displayed as a solid line, yields the SPL (in dB) corresponding to the estimated hearing threshold of the specific user 1. Values for said hearing threshold that have been actually measured within the framework of the estimation are represented by a cross symbol. The underlying true measured hearing threshold of the specific user 1 is displayed as a dotted line $a_{Tr}$ for comparison. Furthermore, as a measure of uncertainty about the true hearing threshold of the specific user 1, the weighted variance $v_k$ $(w_j)$ of the first audiograms A1j at each frequency $f_k$ is represented by respective variance corridors 22, 24, 26 in dash-dotted lines. In each case, the variance corridor 22, 24, 26 is representing the respective weighted variance $v_k$ $(w_j)$ according to the most recent update of the weighting factors $w_j$.

In the left diagram 16, only one observation been made at 8 kHz, i.e., the first frequency $f_1$ of figure 1 has been found at 8 kHz, and the respective hearing threshold th for the specific user 1 has been measured (cross symbol), yielding a result slightly above 80 dB. This knowledge has already been used for performing the first update 10 on the weighting factors wj, such that the corresponding estimated audiogram au as given in eq. (iii) has been calculated, and is displayed in diagram 16 (solid line) along with the weighted variance metric $v_k$ (dash-dotted line) for reference.

It can be seen that the weighted variance metric $v_k$ - as represented by the variance corridor 22 - is smallest for the first frequency $f_1$ = 8 kHz. This is reasonable, as at this frequency there does not exist any uncertainty anymore about the true hearing threshold th of the specific user 1, as it has already been measured. However, at lower frequencies, there still exists a relatively high uncertainty about the validity of the hearing threshold as predicted by the estimated audiogram au.

The weighted variance metric $v_k$ $(w_j)$, according to the first update of $w_j$, is then used to determine, in an iteration step, a distinguished frequency $f_d$, for which this weighted variance metric is largest. This distinguished frequency $f_d$, for the first update of the weighting factors $w_j$ is found at 250 Hz. The next measurement of the hearing threshold th $(f_d)$ of the specific user 1 is performed at said distinguished frequency fd, yielding a result of slightly below 80 dB, as it can be seen in diagram 18. By means of said measured hearing threshold th $(f_d)$ at the distinguished frequency, a second update is performed on the weighting factors $w_j$, on the basis of the difference A1j$(f_d)$ - th$(f_d)$ of the corresponding first audiogram A1j and the measured hearing threshold th at the distinguished frequency. The second update is preferably done in a way that for A1j$(f_d)$ = th$(f_d)$, the weighting factor $w_j$ attains the maximal possible value, and for an increasing difference, A1j$(f_d)$ - th$(f_d)$, the weighting factor $w_j$ decreases more and more, as a result of the second update. Preferably, the second update is also performed on the basis of the difference A1j$(f_1)$ - th$(f_1)$ at the first frequency.

Using the weighting factors $w_j$ according to the second update, the update for the estimated audiogram au is calculated (solid line in diagram 18). As it can be seen from the variance corridor 24 (dash-dotted line) in diagram 18, representing the weighted variance metric $v_k$ with respect to the update for the estimated audiogram au, the maximum uncertainty is now at 2 kHz, and the variance corridor 24 has a maximum width (i.e., distance to the estimated audiogram au) of slightly more than 20 dB, down from the about 40 dB of the variance corridor 22 in diagram 16. Furthermore, the deviation of the estimated audiogram au from the underlying true audiogram $a_{Tr}$ of the specific user 1 has been reduced drastically by the second update, from more than 30 dB at 250 Hz in diagram 16 to about only 5 dB at 1 kHz and 4 kHz in diagram 18.

Diagram 20 shows the estimated audiogram au after a further iteration step, in particular employing a measurement of the hearing threshold th at the next distinguished frequency $f_d$ = 2 kHz as identified from diagram 18. The next (now third) update is performed on the weighting factors wj, and the update of the estimated audiogram au (solid line in diagram 20) is determined in an analogous way as described above. In diagram 20, said updated estimated audiogram au is displayed along with the corresponding variance corridor 26 representing the weighted variance metric (with respect to the updated estimated audiogram au). The maximum width of the variance corridor 20 is further decreased to about 15 dB at 1 kHz.

Further iteration steps may be performed in a similar way. The iteration may be performed a fixed number of runs, or may be stopped after the weighted variance metric $v_k$ (according to the most recent update for the weighting factors wj) falls below a given threshold, or the estimated audiogram au (according to the most recent update for the weighting factors $w_j$) may be visualized and presented to a hearing care professional for decision on whether or not to perform any further iteration steps.

An example for an analytic formula for an iterative updating of the weighting factors $w_j$ may be given in dependence on the following function after m iteration steps (m hearing threshold measurements):

$$Z_j := \sqrt{\sum_{k=1}^{m} \left( A1j(f_k) - th(f_k) \right)^2}$$

(iv)

The quantity $Z_j$ for each first audiogram A1j represents the square-summed deviation from the measured hearing thresholds th $(f_k)$ over the frequencies $f_k$. Then, for the case of equal initial values $I_j = 1$ Vj (or $I_j = 1/N$), the m-th update of the weighting factor $w_j$, i.e., after the m-th hearing threshold measurement, may be given by

$$w_j = \frac{e^{-Z_j}}{\sum_{k}^{N} e^{-Z_j}}.$$

(v)

The relation of eq. (v) is commonly denoted as softmax(-Z) of the vector Z with elements $Z_j$ as given by equation (iv).

In case that not all initial values $I_j$ are equal, the formula for the m-th update (i.e., after hearing threshold measurements at m different frequencies) may be adjusted to

$$w_j = \frac{I_j}{m+1} + \frac{m}{m+1} \cdot \frac{e^{-Z_j}}{\sum_{k}^{N} e^{-Z_j}},$$

wherein the denominator m+1 ensures that for each iteration step m, the initial value $I_j$ has less contribution to the update of the weighting factor $w_j$.

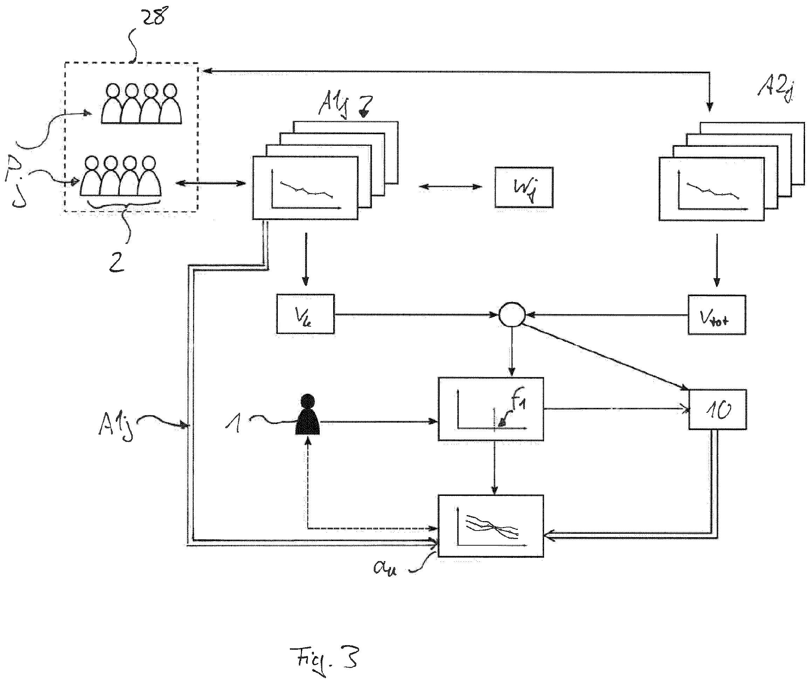

In figure 3, a schematic block diagram is shown, said block diagram displaying the workflow of an extension to the method shown in figure 1. As in figure 1, a first plurality of first audiograms A1j is provided from a database, each of which corresponding to a hearing impaired person $P_j$ out of a first plurality 2 of persons. Now, also second plurality of second audiograms A2j is provided from the database, each of which corresponding to a hearing impaired person $P_j$ out of a second plurality 28 of persons. The first plurality 2 of persons in this case is a true subset of the second plurality 28 of persons, i.e., each person $P_j$ in the first plurality 2 is also part of the second plurality 28, but there are persons $P_j$ in the second plurality 28 that are excluded from the first plurality 2.

In the embodiment of figure 3, the second audiograms A2j are given by "normal" audiograms that have been measured, e.g., by means of an audiometer, while the first audiograms A1j are given by in-situ audiograms, measured with a hearing aid located in the respective ear during measurement. This corresponds to the typical situation that "normal" audiograms, i.e., the second audiograms A2j, are much easier available (as they get measured more often), while data on in-situ audiograms, i.e., the first audiograms A1j, may be somewhat scarce.

Now, the same way as described in figure 1, the weighted variance metric $v_k$ is determined for the first audiograms A1j at each frequency $f_k$ out of the first set $s_1$ of frequencies $f_k$, based on the initial values $I_j$ of the respective weighting factors $w_j$ associated to the first audiograms A1j. In a similar way, the total variance $v_{tot}$ is calculated for the second audiograms A2j at each frequency $f_k$ out of the first set $s_1$ of frequencies. In case that for the second audiograms A2j, there exist respective second weighting factors with non-trivial initial values, also a weighted variance metric based on said initial values of said weighting factors may be calculated for the second plurality of second audiograms A2j.

The total variance $v_{tot}$ of the second plurality of second audiograms A2j is now compared to the corresponding weighted variance metric $v_k$ of the first plurality of first audiograms A1j at each frequency $f_k$. In case that the difference remains below a given threshold for all frequencies $f_k$, this means that the statistical information of the second audiograms A2j (the "normal" audiograms) is not too different from the statistical information contained in the first audiograms A1j (the in-situ audiograms), so that the determination of the first frequency $f_1$ and/or the update of the weighting factors $w_j$ for the first audiograms A1j may be performed also on basis of the second plurality of second audiograms A2j, and in particular on their total variance vtot.

The estimated audiogram au of the specific user is then determined from the first audiograms A1j and the

corresponding weighting factors $w_j$ for the first audiograms A1j, with the values resulting from the first update 10, according to equation (iii), as described in figure 1.

Even though the invention has been illustrated and described in detail with help of a preferred embodiment example, the invention is not restricted by this example. Other variations can be derived by a person skilled in the art without leaving the extent of protection of this invention.

Reference numeral

| 1 | specific user |
| 2 | first plurality (of persons) |
| 4 | variance corridor |
| 6 | diagram |
| 8 | width |
| 10 | first update |
| 12 | diagram |
| 14 | variance corridor |
| 16 | diagram |
| 18 | diagram |
| 20 | diagram |
| 22 | variance corridor |
| 24 | variance corridor |
| 26 | variance corridor |
| 28 | second plurality (of persons) |

| $a_{Tr}$ | true audiogram |
| $a_u$ | estimated audiogram |
| A1j | first audiogram |
| A2j | second audiogram |
| $f_1$ | first frequency |
| $f_d$ | distinguished frequency |
| $f_k$ | frequency |
| $I_j$ | initial value |
| $P_j$ | (hearing impaired) person |
| $s_1$ | first set (of frequencies) |
| th | (measured) hearing threshold (at a given frequency) |
| $v_k$ | weighted variance metric |
| $v_{tot}$ | total variance |
| $w_j$ | weighting factor |

**Claims**

1. A hearing estimation system comprising a computerized device and an acoustic output transducer, wherein the computerized device comprises or is operationally connected to the acoustic output transducer and wherein the computerized device comprises a graphical user interface, a program storage for storing an executable program and a processor for executing said program to perform a method, comprising the steps of:

   a) receiving a first plurality of first audiograms (A1j) from a corresponding first plurality (2) of persons ($P_j$);
   b) providing, for each of the first audiograms (A1j), a weighting factor ($w_j$) and an initial value ($I_j$) of said weighting factor ($w_j$);
   c) selecting a first frequency ($f_1$), out of a first set ($s_1$) of frequencies ($f_k$);
   d) measuring a hearing threshold (th) of a specific user (1) at said first frequency ($f_1$) using said acoustic output transducer;
   e) performing a first update (10) on each of said weighting factors ($w_j$) corresponding to said first plurality of audiograms (A1j) in dependence on the difference between the value of the respective first audiogram (A1j) and said measured hearing threshold (th) of said specific user (1) at said first frequency ($f_1$), wherein said weighting factors ($w_j$) decreases with an increasing difference between said value of the respective first audiogram (A1j) and said measured hearing threshold (th) of said specific user (1) at said first frequency ($f_1$); and

f) determining an estimated audiogram ($a_u$) for said specific user (1) as a weighted mean of said first plurality of first audiograms (A1j) based on said weighting factors ($w_j$), according to their respective first update (10).

2. The hearing estimation system according to claim 1, wherein said method comprises a further step of:

c') determining, at each of said first set ($s_1$) of frequencies ($f_k$), a weighted variance metric ($v_k$) for said first plurality of first audiograms (A1j), in dependence of said weighting factors ($w_j$),

wherein the first frequency ($f_1$) is selected out of said first set ($s_1$) of frequencies ($f_k$) by a determination in dependence on the frequency out of a first frequency range for which the weighted variance metric ($v_k$) is largest.

3. The hearing estimation system according to claim 1 or claim 2, wherein said method comprises an iteration of the following steps:

g) determining, at several frequencies ($f_k$) out of said first set ($s_1$) of frequencies ($f_k$), a weighted variance metric ($v_k$) for said first plurality of first audiograms (A1j) in dependence on said weighting factors ($w_j$), according to their most recent update, respectively;

h) determining a distinguished frequency ($f_d$) out of said several frequencies ($f_k$), in dependence on the frequency out of a second frequency range for which said weighted variance metric ($v_k$) is largest;

i) measuring a hearing threshold (th) of said specific user (1) at said distinguished frequency ($f_d$);

j) performing an update on each of said weighting factors ($w_j$) corresponding to said first plurality of first audiograms (A1j) also in dependence on the difference between the value of the respective first audiogram (A1j) and said measured hearing threshold (th) of said specific user (1) at said distinguished frequency ($f_d$), respectively;

and, after finishing said iteration, further comprising the step of

f') determining the estimated audiogram ($a_u$) for said specific user (1) as a weighted mean of said first plurality of first audiograms (A1j), using said weighting factors ($w_j$) according to their most recent update, respectively, for said weighted mean.

4. The hearing estimation system according to claim 3, wherein the iteration is finished after completion of a given number of iteration runs or when the weighted variance metric ($v_k$) for said first plurality of first audiograms (A1j) or a variance for said first plurality of first audiograms (A1j) falls below a given first threshold.

5. The hearing estimation system according to claim 3, wherein, after each iteration run,

- the estimated audiogram ($a_u$) for said specific user (1) is determined as a weighted mean of said first plurality of first audiograms (A1j), using said weighting factors ($w_j$) according to their most recent update, respectively, for said weighted mean,

- the estimated audiogram ($a_u$) is visualized using said graphical user interface, and

- said visualized estimated audiogram ($a_u$) is presented to a hearing care professional for decision about stopping the iteration.

6. The hearing estimation system according to one of the preceding claims, wherein for each weighting factor ($w_j$), the initial value ($I_j$) is chosen in dependence on a demographic similarity of the specific person (1) with the person ($P_j$) corresponding to the first audiogram (A1j) for which the weighting factor ($w_j$) is being provided.

7. The hearing estimation system according to one of the preceding claims, wherein the first plurality of first audiograms (A1j) is chosen as a subset out of a multiplicity of first audiograms (A1j) from a corresponding multiplicity of persons ($P_j$) in dependence on a demographic similarity of the specific user (1) with a person ($P_j$) from said multiplicity corresponding to a respective first audiogram (A1j).

8. The hearing estimation system according to one of the preceding claims, wherein a starting sound pressure level for measuring the hearing threshold (th) of said specific user (1) at a certain frequency ($f_k$) is chosen in dependence on the estimated audiogram ($a_u$) and/or the weighted variance metric ($v_k$) at said certain frequency ($f_k$), using the weighting factors ($w_j$) according to their most recent update, respectively.

9. The hearing estimation system according to one of the preceding claims, wherein a continuous fit is performed on the weighted variance metric ($v_k$) for all frequencies ($f_k$) in the interval spanned by said first set ($s_1$) of frequencies ($f_k$), and

wherein the first frequency ($f_1$) is determined from said continuous fit.

10. The hearing estimation system according to claim 9,
wherein in order to obtain a value of a first audiogram (A1j) at the first frequency ($f_1$), an interpolation and/or a continuous fit is performed on basis of said first audiogram's (A1j) values at the frequencies ($f_k$) of said first set ($s_1$) of frequencies ($f_k$).

11. The hearing estimation system according to one of the preceding claims, wherein said method comprises the further steps of:

   - providing a second plurality of second audiograms (A2j) from a corresponding second plurality (28) of persons ($P_j$), wherein each person ($P_j$) out of the first plurality (2) is also comprised in the second plurality (28) of persons ($P_j$);
   - providing, for each of the second audiograms (A2j), a value of a weighting factor;
   - determining, at each of a second set of frequencies, a weighted variance metric for said second plurality of second audiograms (A2j), in dependence of said weighting factors, wherein the second set of frequencies is a subset said first set ($s_1$) of frequencies ($f_k$); and
   - determining said first frequency ($f_1$) and/or said distinguished frequency ($f_d$) for the first plurality of first audiograms (A1j) and/or performing said first update (10) on the weighting factors ($w_j$) corresponding to the first audiograms (A1j) also based on the weighted variance metric for said second plurality of second audiograms (A2j).

12. The hearing estimation system according to claim 11,

   wherein the first and second pluralities (2, 28) comprise the same persons ($P_j$), and
   wherein for each person ($P_j$) out of said first and second pluralities (2, 28), respectively, the corresponding first and second audiogram (A1j, A2j) are representing the hearing at either of the two ears.

13. The hearing estimation system according to claim 11,

   wherein each of the first audiograms (A1j) is measured as an in-situ audiogram in the presence of a hearing aid at the respective ear of the corresponding person ($P_j$) out of the first plurality (2), and
   wherein each of the second audiograms (A2j) is measured in the absence of a hearing aid at the respective ear of the corresponding person ($P_j$) out of the second plurality (28).

14. A non-transitory computer readable medium carrying instructions which, when executed by the processor of a hearing estimation system according to claim 1, cause the following method steps to be performed:

   a) receiving a first plurality of first audiograms (A1j) from a corresponding first plurality (2) of persons (Pj);
   b) providing, for each of the first audiograms (A1j), a weighting factor (wj) and an initial value (Ij) of said weighting factor (wj);
   c) selecting a first frequency (f1), out of a first set (s1) of frequencies (fk);
   d) using the electro-acoustical transducer of the hearing estimation system to measure a hearing threshold (th) of a specific user (1) at said first frequency (f1);
   e) performing a first update (10) on each of said weighting factors (wj) corresponding to said first plurality of audiograms (A1j) in dependence on the difference between the value of the respective first audiogram (A1j) and said measured hearing threshold (th) of said specific user (1) at said first frequency ($f_1$), wherein said weighting factors ($w_j$) decreases with an increasing difference between said value of the respective first audiogram (A1j) and said measured hearing threshold (th) of said specific user (1) at said first frequency ($f_1$), and
   f) determining an estimated audiogram (au) for said specific user (1) as a weighted mean of said first plurality of first audiograms (A1j) based on said weighting factors (wj), according to their respective first update (10).

15. The non-transitory computer readable medium according to claim 14 causing the following further method step to be performed:

   c') determining, at each of said first set ($s_1$) of frequencies ($f_k$), a weighted variance metric ($v_k$) for said first plurality of first audiograms (A1j), in dependence of said weighting factors ($w_j$),
   wherein the first frequency ($f_1$) is selected out of said first set ($s_1$) of frequencies ($f_k$) by a determination in dependence on the frequency out of a first frequency range for which the weighted variance metric ($v_k$) is largest.

**Patentansprüche**

1. Hörschätzungssystem, welches eine computerisierte Vorrichtung und einen akustischen Ausgangswandler umfasst, wobei die computerisierte Vorrichtung den akustischen Ausgangswandler umfasst oder mit ihm wirkverbunden ist und wobei die computerisierte Vorrichtung eine grafische Benutzeroberfläche, einen Programmspeicher zum Speichern eines ausführbaren Programms und einen Prozessor zum Ausführen des Programms umfasst, um ein Verfahren durchzuführen, welches die folgenden Schritte umfasst:

   a) Empfangen einer ersten Vielzahl von ersten Audiogrammen (A1j) von einer entsprechenden ersten Vielzahl (2) von Personen ($P_j$);
   b) Bereitstellen eines Gewichtungsfaktors ($w_j$) und eines Anfangswerts ($I_j$) des Gewichtungsfaktors ($w_j$) für jedes der ersten Audiogramme (A1j);
   c) Auswählen einer ersten Frequenz ($f_1$) aus einer ersten Menge ($s_1$) von Frequenzen ($f_k$);
   d) Messen einer Hörschwelle (th) eines betreffenden Benutzers (1) bei der ersten Frequenz ($f_1$) unter Verwendung des akustischen Ausgangswandlers;
   e) Durchführen einer ersten Aktualisierung (10) jedes der Gewichtungsfaktoren ($w_j$), welche der ersten Vielzahl von Audiogrammen (A1j) entsprechen, in Abhängigkeit von der Differenz zwischen dem Wert des jeweiligen ersten Audiogramms (A1j) und der gemessenen Hörschwelle (th) des betreffenden Benutzers (1) bei der ersten Frequenz ($f_1$), wobei die Gewichtungsfaktoren ($w_j$) mit zunehmender Differenz zwischen dem Wert des jeweiligen ersten Audiogramms (A1j) und der gemessenen Hörschwelle (th) des betreffenden Benutzers (1) bei der ersten Frequenz ($f_1$) abnehmen; und
   f) Bestimmen eines geschätzten Audiogramms ($a_u$) für den betreffenden Benutzer (1) als einen gewichteten Mittelwert der ersten Vielzahl von ersten Audiogrammen (A1j) basierend auf den Gewichtungsfaktoren ($w_j$) gemäß ihrer jeweiligen ersten Aktualisierung (10).

2. Hörschätzungssystem nach Anspruch 1, wobei das Verfahren einen folgenden weiteren Schritt umfasst:

   c') Bestimmen einer gewichteten Varianzmetrik ($v_k$) für jede der ersten Menge ($s_1$) von Frequenzen ($f_k$) für die erste Vielzahl von ersten Audiogrammen (A1j) in Abhängigkeit von den Gewichtungsfaktoren ($w_j$),
   wobei die erste Frequenz ($f_1$) aus der ersten Menge ($s_1$) von Frequenzen ($f_k$) durch eine Bestimmung in Abhängigkeit von der Frequenz aus einem ersten Frequenzbereich ausgewählt wird, für welchen die gewichtete Varianzmetrik ($v_k$) am größten ist.

3. Hörschätzungssystem nach Anspruch 1 oder Anspruch 2, wobei das Verfahren eine Iteration der folgenden Schritte umfasst:

   g) Bestimmen einer gewichteten Varianzmetrik ($v_k$) für mehrere Frequenzen ($f_k$) aus der ersten Menge ($s_1$) von Frequenzen ($f_k$) für die erste Vielzahl von ersten Audiogrammen (A1j) in Abhängigkeit von den Gewichtungsfaktoren ($w_j$) gemäß ihrer jeweils aktuellsten Aktualisierung;
   h) Bestimmen einer eindeutigen Frequenz ($f_d$) aus den mehreren Frequenzen ($f_k$) in Abhängigkeit von der Frequenz aus einem zweiten Frequenzbereich, für welche die gewichtete Varianzmetrik ($v_k$) am größten ist;
   i) Messen einer Hörschwelle (th) eines betreffenden Benutzers (1) bei der eindeutigen Frequenz ($f_d$);
   j) Durchführen einer Aktualisierung jedes der Gewichtungsfaktoren ($w_j$), welche den ersten Vielzahl von ersten Audiogrammen (A1j) entsprechen, auch in Abhängigkeit von der Differenz zwischen dem Wert des jeweiligen ersten Audiogramms (A1j) und der gemessenen Hörschwelle (th) des betreffenden Benutzers (1) bei der jeweils eindeutigen Frequenz ($f_d$),
   und, nach Abschluss dieser Iteration, weiter den folgenden Schritt umfassend:
   f') Bestimmen des geschätzten Audiogramms ($a_u$) für den betreffenden Benutzer (1) als einen gewichteten Mittelwert der ersten Vielzahl von ersten Audiogrammen (A1j) unter Verwendung der Gewichtungsfaktoren ($w_j$) gemäß ihrer jeweils aktuellsten Aktualisierung für den gewichteten Mittelwert.

4. Hörschätzungssystem nach Anspruch 3, wobei die Iteration nach Abschluss einer vorgegebenen Anzahl von Iterationsläufen oder, wenn die gewichtete Varianzmetrik ($v_k$) für die erste Vielzahl von ersten Audiogrammen (A1j) oder eine Varianz für die erste Vielzahl von ersten Audiogrammen (A1j) unter einen vorgegebenen ersten Schwellenwert fällt, beendet ist.

**5.** Hörschätzungssystem nach Anspruch 3, wobei nach jedem Iterationslauf

- das geschätzte Audiogramm ($a_u$) für den betreffenden Benutzer (1) als ein gewichteter Mittelwert der ersten Vielzahl von ersten Audiogrammen (A1j) unter Verwendung der Gewichtungsfaktoren ($w_j$) gemäß ihrer jeweils aktuellsten Aktualisierung für den gewichteten Mittelwert bestimmt wird,
- das geschätzte Audiogramm ($a_u$) unter Verwendung der grafischen Benutzeroberfläche visualisiert wird, und
- das visualisierte geschätzte Audiogramm ($a_u$) einem Hörgeräteakustiker zur Entscheidung über den Abbruch der Iteration vorgelegt wird.

**6.** Hörschätzungssystem nach einem der vorstehenden Ansprüche, wobei für jeden Gewichtsfaktor ($w_j$) der Anfangswert ($I_j$) in Abhängigkeit von einer demografischen Ähnlichkeit der betreffenden Person (1) mit der Person ($P_j$) gewählt wird, welche dem ersten Audiogramm (A1j) entspricht, für welches der Gewichtungsfaktor ($w_j$) bereitgestellt wird.

**7.** Hörschätzungssystem nach einem der vorstehenden Ansprüche, wobei die erste Vielzahl von ersten Audiogrammen (A1j) als eine Teilmenge aus einer Multiplizität von ersten Audiogrammen (A1j) aus einer entsprechenden Multiplizität von Personen ($P_j$) in Abhängigkeit von einer demografischen Ähnlichkeit des betreffenden Benutzers (1) mit einer Person ($P_j$) aus dieser Multiplizität, welche einem jeweiligen ersten Audiogramm (A1j) entspricht, gewählt wird.

**8.** Hörschätzungssystem nach einem der vorstehenden Ansprüche, wobei ein Anfangsschalldruckpegel zum Messen der Hörschwelle (th) des betreffenden Benutzers (1) bei einer bestimmten Frequenz ($f_k$) in Abhängigkeit von dem geschätzten Audiogramm ($a_u$) und/oder der gewichteten Varianzmetrik ($v_k$) bei der bestimmten Frequenz ($f_k$) unter Verwendung der Gewichtungsfaktoren ($w_j$) gemäß ihrer jeweils aktuellsten Aktualisierung gewählt wird.

**9.** Hörschätzungssystem nach einem der vorstehenden Ansprüche, wobei eine kontinuierliche Anpassung der gewichteten Varianzmetrik ($v_k$) für alle Frequenzen ($f_k$) in dem Intervall, welches von der ersten Menge ($s_1$) von Frequenzen ($f_k$) aufgespannt wird, durchgeführt wird, und wobei die erste Frequenz ($f_1$) aus dieser kontinuierlichen Anpassung bestimmt wird.

**10.** Hörschätzungssystem nach Anspruch 9,
wobei zum Erhalten eines Wertes eines ersten Audiogramms (A1j) bei der ersten Frequenz ($f_1$) eine Interpolation und/oder eine kontinuierliche Anpassung basierend auf den Werten des ersten Audiogramms (A1j) bei den Frequenzen ($f_k$) der ersten Menge ($s_1$) von Frequenzen ($f_k$) durchgeführt wird.

**11.** Hörschätzungssystem nach einem der vorstehenden Ansprüche, wobei das Verfahren die folgenden weiteren Schritte umfasst:

- Bereitstellen einer zweiten Vielzahl von zweiten Audiogrammen (A2j) aus einer entsprechenden zweiten Vielzahl (28) von Personen ($P_j$), wobei jede Person ($P_j$) aus der ersten Vielzahl (2) auch in der zweiten Vielzahl (28) von Personen ($P_j$) enthalten ist;
- Bereitstellen eines Werts eines Gewichtungsfaktors für jedes der zweiten Audiogramme (A2j);
- Bestimmen einer gewichteten Varianzmetrik für jede einer zweiten Menge von Frequenzen für die zweite Vielzahl von zweiten Audiogrammen (A2j) in Abhängigkeit von den Gewichtungsfaktoren, wobei die zweite Menge von Frequenzen eine Teilmenge der ersten Menge ($s_1$) von Frequenzen ($f_k$) ist; und
- Bestimmen der ersten Frequenz ($f_1$) und/oder der eindeutigen Frequenz ($f_d$) für die erste Vielzahl von ersten Audiogrammen (A1j) und/oder Durchführen der ersten Aktualisierung (10) an den Gewichtungsfaktoren ($w_j$), welche den ersten Audiogrammen (A1j) entsprechen, auch basierend auf der gewichteten Varianzmetrik für die zweite Vielzahl von zweiten Audiogrammen (A2j).

**12.** Hörschätzungssystem nach Anspruch 11,

wobei die erste und zweite Vielzahl (2, 28) dieselben Personen ($P_j$) umfassen, und
wobei für jede Person ($P_j$) aus der ersten und zweiten Vielzahl (2, 28) jeweils das entsprechende erste und zweite Audiogramm (A1j, A2j) das Hörvermögen an einem der beiden Ohren darstellt.

**13.** Hörschätzungssystem nach Anspruch 11,

wobei jedes der ersten Audiogramme (A1j) als In-situ-Audiogramm in Anwesenheit eines Hörgeräts am jeweiligen Ohr der entsprechenden Person ($P_j$) aus der ersten Vielzahl (2) gemessen wird, und
wobei jedes der zweiten Audiogramme (A2j) in Abwesenheit eines Hörgeräts am jeweiligen Ohr der entsprechenden Person ($P_j$) aus der zweiten Vielzahl (28) gemessen wird.

14. Nichtflüchtiges computerlesbares Medium, welches Anweisungen enthält, welche, wenn sie von dem Prozessor eines Hörschätzungssystems nach Anspruch 1 ausgeführt werden, veranlassen, dass die folgenden Verfahrensschritte ausgeführt werden:

a) Empfangen einer ersten Vielzahl von ersten Audiogrammen (A1j) von einer entsprechenden ersten Vielzahl (2) von Personen (Pj);
b) Bereitstellen eines Gewichtungsfaktors (wj) und eines Anfangswerts (lj) dieses Gewichtungsfaktors (wj) für jedes der ersten Audiogramme (A1j);
c) Auswählen einer ersten Frequenz (f1) aus einer ersten Menge (s1) von Frequenzen (fk);
d) Verwenden des elektroakustischen Wandlers des Hörschätzungssystems zum Messen der Hörschwelle (th) eines betreffenden Benutzers (1) bei der ersten Frequenz (f1);
e) Durchführen einer ersten Aktualisierung (10) jedes der Gewichtungsfaktoren (wj), welche der ersten Vielzahl von Audiogrammen (A1j) entsprechen, in Abhängigkeit von der Differenz zwischen dem Wert des jeweiligen ersten Audiogramms (A1j) und der gemessenen Hörschwelle (th) des betreffenden Benutzers (1) bei der ersten Frequenz (f1),
wobei die Gewichtungsfaktoren (wj) mit zunehmender Differenz zwischen dem Wert des jeweiligen ersten Audiogramms (A1j) und der gemessenen Hörschwelle (th) des betreffenden Benutzers (1) bei der ersten Frequenz ($f_1$) abnehmen; und
f) Bestimmen eines geschätzten Audiogramms (au) für den betreffenden Benutzer (1) als einen gewichteten Mittelwert der ersten Vielzahl von ersten Audiogrammen (A1j) basierend auf den Gewichtungsfaktoren (wj) gemäß ihrer jeweiligen ersten Aktualisierung (10).

15. Nichtflüchtiges computerlesbares Medium nach Anspruch 14, welches veranlasst, dass die folgenden Verfahrensschritte ausgeführt werden:

c') Bestimmen einer gewichteten Varianzmetrik ($v_k$) für jede der ersten Menge ($s_1$) von Frequenzen ($f_k$) für die erste Vielzahl von ersten Audiogrammen (A1j) in Abhängigkeit von den Gewichtungsfaktoren ($w_j$),
wobei die erste Frequenz ($f_1$) aus der ersten Menge ($s_1$) von Frequenzen ($f_k$) durch eine Bestimmung in Abhängigkeit von der Frequenz aus einem ersten Frequenzbereich ausgewählt wird, für welchen die gewichtete Varianzmetrik ($v_k$) am größten ist.

## Revendications

1. Système d'estimation d'audition comprenant un dispositif informatisé et un transducteur de sortie acoustique, dans lequel le dispositif informatisé comprend ou est connecté de manière opérationnelle au transducteur de sortie acoustique et dans lequel le dispositif informatisé comprend une interface utilisateur graphique, un stockage de programme pour stocker un programme exécutable et un processeur pour exécuter ledit programme pour réaliser un procédé comprenant les étapes consistant à :

a) recevoir une première pluralité de premiers audiogrammes (A1j) en provenance d'une première pluralité correspondante (2) de personnes ($P_j$) ;
b) fournir, pour chacun des premiers audiogrammes (A1j), un facteur de pondération ($w_j$) et une valeur initiale ($l_j$) dudit facteur de pondération ($w_j$) ;
c) sélectionner une première fréquence ($f_1$) parmi un premier ensemble ($s_1$) de fréquences ($f_k$) ;
d) mesurer un seuil d'audition (th) d'un utilisateur spécifique (1) à ladite première fréquence ($f_1$) en utilisant ledit transducteur de sortie acoustique ;
e) réaliser une première mise à jour (10) sur chacun desdits facteurs de pondération ($w_j$) correspondant à ladite première pluralité d'audiogrammes (A1j) en fonction de la différence entre la valeur du premier audiogramme respectif (A1j) et ledit seuil d'audition mesuré (th) dudit utilisateur spécifique (1) à ladite première fréquence ($f_1$),
dans lequel lesdits facteurs de pondération ($w_j$) diminuent avec une augmentation de la différence entre ladite valeur du premier audiogramme respectif (A1j) et ledit seuil d'audition mesuré (th) dudit utilisateur spécifique (1) à ladite première fréquence ($f_1$) ; et
f) déterminer un audiogramme estimé ($a_u$) pour ledit utilisateur spécifique (1) comme une moyenne pondérée de ladite première pluralité de premiers audiogrammes (A1j) sur la base desdits facteurs de pondération ($w_j$), selon leur première mise à jour respective (10).

2. Système d'estimation d'audition selon la revendication 1, dans lequel ledit procédé comprend une étape supplémentaire consistant à :

c') déterminer, pour chacun dudit premier ensemble ($s_1$) de fréquences ($f_k$), une métrique de variance pondérée ($v_k$) pour ladite première pluralité de premiers audiogrammes (A1j), en fonction desdits facteurs de pondération ($w_j$),

dans lequel la première fréquence ($f_1$) est sélectionnée parmi ledit premier ensemble ($s_1$) de fréquences ($f_k$) par une détermination en fonction de la fréquence parmi une première plage de fréquences pour laquelle la métrique de variance pondérée ($v_k$) est la plus grande.

3. Système d'estimation d'audition selon la revendication 1 ou la revendication 2, dans lequel ledit procédé comprend une itération des étapes suivantes :

g) déterminer, au niveau de plusieurs fréquences ($f_k$) parmi ledit premier ensemble ($s_1$) de fréquences ($f_k$), une métrique de variance pondérée ($v_k$) pour ladite première pluralité de premiers audiogrammes (A1j) en fonction desdits facteurs de pondération ($w_j$), selon leur mise à jour la plus récente, respectivement ;

h) déterminer une fréquence notable ($f_d$) parmi lesdites plusieurs fréquences ($f_k$), en fonction de la fréquence parmi une seconde plage de fréquences pour laquelle ladite métrique de variance pondérée ($v_k$) est la plus grande ;

i) mesurer un seuil d'audition (th) dudit utilisateur spécifique (1) à ladite fréquence notable ($f_d$) ;

j) réaliser une mise à jour sur chacun desdits facteurs de pondération ($w_j$) correspondant à ladite première pluralité de premiers audiogrammes (A1j) également en fonction de la différence entre la valeur du premier audiogramme respectif (A1j) et ledit seuil d'audition mesuré (th) dudit utilisateur spécifique (1) à ladite fréquence notable ($f_d$), respectivement ;

et, après avoir terminé ladite itération, comprenant en outre l'étape consistant à

f') déterminer l'audiogramme estimé ($a_u$) pour ledit utilisateur spécifique (1) comme une moyenne pondérée de ladite première pluralité de premiers audiogrammes (A1j), en utilisant lesdits facteurs de pondération ($w_j$) selon leur mise à jour la plus récente, respectivement, pour ladite moyenne pondérée.

4. Système d'estimation d'audition selon la revendication 3, dans lequel l'itération est terminée après l'achèvement d'un nombre donné d'itérations ou lorsque la métrique de variance pondérée ($v_k$) pour ladite première pluralité de premiers audiogrammes (A1j) ou une variance pour ladite première pluralité de premiers audiogrammes (A1j) tombe en dessous d'un premier seuil donné.

5. Système d'estimation d'audition selon la revendication 3, dans lequel, après chaque itération,

- l'audiogramme estimé ($a_u$) pour ledit utilisateur spécifique (1) est déterminé comme une moyenne pondérée de ladite première pluralité de premiers audiogrammes (A1j), en utilisant lesdits facteurs de pondération ($w_j$) selon leur mise à jour la plus récente, respectivement, pour ladite moyenne pondérée,

- l'audiogramme estimé ($a_u$) est visualisé en utilisant ladite interface utilisateur graphique, et

- ledit audiogramme estimé visualisé ($a_u$) est présenté à un audioprothésiste afin qu'il prenne la décision d'arrêter ou non l'itération.

6. Système d'estimation d'audition selon l'une des revendications précédentes, dans lequel pour chaque facteur de pondération ($w_j$), la valeur initiale ($I_j$) est choisie en fonction d'une similitude démographique de la personne spécifique (1) avec la personne ($P_j$) correspondant au premier audiogramme (A1j) pour lequel le facteur de pondération ($w_j$) est fourni.

7. Système d'estimation d'audition selon l'une des revendications précédentes, dans lequel la première pluralité de premiers audiogrammes (A1j) est choisie comme sous-ensemble parmi une multiplicité de premiers audiogrammes (A1j) en provenance d'une multiplicité correspondante de personnes ($P_j$) en fonction d'une similitude démographique de l'utilisateur spécifique (1) avec une personne ($P_j$) de ladite multiplicité correspondant à un premier audiogramme respectif (A1j).

8. Système d'estimation d'audition selon l'une des revendications précédentes, dans lequel un niveau de pression acoustique initial pour mesurer le seuil d'audition (th) dudit utilisateur spécifique (1) à une certaine fréquence ($f_k$) est choisi en fonction de l'audiogramme estimé ($a_u$) et/ou de la métrique de variance pondérée ($v_k$) à ladite certaine fréquence ($f_k$), en utilisant les facteurs de pondération ($w_j$) selon leur mise à jour la plus récente, respectivement.

9. Système d'estimation d'audition selon l'une des revendications précédentes, dans lequel un ajustement continu est réalisé sur la métrique de variance pondérée ($v_k$) pour toutes les fréquences ($f_k$) dans l'intervalle couvert par ledit premier ensemble ($s_1$) de fréquences ($f_k$) et dans lequel la première fréquence ($f_1$) est déterminée à partir dudit ajustement continu.

10. Système d'estimation d'audition selon la revendication 9,
dans lequel afin d'obtenir une valeur d'un premier audiogramme (A1j) à la première fréquence ($f_1$), une interpolation et/ou un ajustement continu sont réalisés sur la base des valeurs dudit premier audiogramme (A1j) aux fréquences ($f_k$) dudit premier ensemble ($s_1$) de fréquences ($f_k$).

11. Procédé d'estimation d'audition selon l'une des revendications précédentes, dans lequel ledit procédé comprend outre les étapes consistant à :

- fournir une seconde pluralité de seconds audiogrammes (A2j) en provenance d'une seconde pluralité correspondante (28) de personnes ($P_j$), dans lequel chaque personne ($P_j$) parmi la première pluralité (2) est également comprise dans la seconde pluralité (28) de personnes ($P_j$) ;
- fournir, pour chacun des seconds audiogrammes (A2j), une valeur d'un facteur de pondération ;
- déterminer, à chacun d'un second ensemble de fréquences, une métrique de variance pondérée pour ladite seconde pluralité de seconds audiogrammes (A2j), en fonction desdits facteurs de pondération, dans lequel le second ensemble de fréquences est un sous-ensemble dudit premier ensemble ($s_1$) de fréquences ($f_k$) ; et
- déterminer ladite première fréquence ($f_1$) et/ou ladite fréquence notable ($f_d$) pour la première pluralité de premiers audiogrammes (A1j) et/ou réaliser ladite première mise à jour (10) sur les facteurs de pondération ($w_j$) correspondant aux premiers audiogrammes (A1j) également sur la base de la métrique de variance pondérée pour ladite seconde pluralité de seconds audiogrammes (A2j).

12. Système d'estimation d'audition selon la revendication 11,

dans lequel les première et seconde pluralités (2, 28) comprennent les mêmes personnes ($P_j$), et
dans lequel pour chaque personne ($P_j$) parmi lesdites première et seconde pluralités (2, 28), respectivement, les premier et second audiogrammes correspondants (A1j, A2j) représentent l'audition à l'une ou l'autre des deux oreilles.

13. Système d'estimation d'audition selon la revendication 11,

dans lequel chacun des premiers audiogrammes (A1j) est mesuré comme un audiogramme in situ en présence d'une aide auditive à l'oreille respective de la personne correspondante ($P_j$) parmi la première pluralité (2), et
dans lequel chacun des seconds audiogrammes (A2j) est mesuré en l'absence d'une aide auditive à l'oreille respective de la personne correspondante ($P_j$) parmi la seconde pluralité (28).

14. Support non transitoire lisible par ordinateur portant des instructions qui, lorsqu'elles sont exécutées par le processeur d'un système d'estimation d'audition selon la revendication 1, amènent les étapes de procédé suivantes à être réalisées :

a) réception d'une première pluralité de premiers audiogrammes (A1j) en provenance d'une première pluralité correspondante (2) de personnes (Pj) ;
b) fourniture, pour chacun des premiers audiogrammes (A1j), d'un facteur de pondération (wj) et d'une valeur initiale (Ij) dudit facteur de pondération (wj) ;
c) sélection d'une première fréquence (f1), parmi un premier ensemble (s1) de fréquences (fk) ;
d) utilisation du transducteur électroacoustique du système d'estimation d'audition pour mesurer un seuil d'audition (th) d'un utilisateur spécifique (1) à ladite première fréquence (f1) ;
e) réalisation d'une première mise à jour (10) sur chacun desdits facteurs de pondération (wj) correspondant à ladite première pluralité d'audiogrammes (A1j) en fonction de la différence entre la valeur du premier audiogramme respectif (A1j) et ledit seuil d'audition mesuré (th) dudit utilisateur spécifique (1) à ladite première fréquence (f1),

dans lequel lesdits facteurs de pondération (wj) diminuent avec une augmentation de la différence entre ladite valeur du premier audiogramme respectif (A1j) et ledit seuil d'audition mesuré (th) dudit utilisateur spécifique (1) au niveau de ladite première fréquence ($f_1$) ; et

f) détermination d'un audiogramme estimé (au) pour ledit utilisateur spécifique (1) comme une moyenne pondérée de ladite première pluralité de premiers audiogrammes (A1j) sur la base desdits facteurs de pondération (wj), selon leur première mise à jour respective (10).

15. Support non transitoire lisible par ordinateur selon la revendication 14, entraînant la réalisation de l'étape de procédé supplémentaire suivante :

c') détermination, au niveau de chacune dudit premier ensemble ($s_1$) de fréquences ($f_k$), d'une métrique de variance pondérée ($v_k$) pour ladite première pluralité de premiers audiogrammes (A1j), en fonction desdits facteurs de pondération ($w_j$),

dans lequel la première fréquence ($f_1$) est sélectionnée parmi ledit premier ensemble ($s_1$) de fréquences ($f_k$) par une détermination en fonction de la fréquence parmi une première plage de fréquences pour laquelle la métrique de variance pondérée ($v_k$) est la plus grande.

Fig. 1

EP 4 322 850 B1

Fig. 2

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019195866 A1
- US 2016345111 A1
- WO 2020214482 A1

**Non-patent literature cited in the description**

- **OÊZDAMAR Ö et al.** Classification of audiograms by sequential testing using a dynamic Bayesian procedure. *The Journal of the Acoustical Society of America*, 01 January 1990